# EUROPÄISCHE PATENTSCHRIFT

(11) **EP 1 066 807 B1**
(45) Veröffentlichungstag und Bekanntmachung des Hinweises auf die Patenterteilung: **10.03.2004**
(21) Anmeldenummer: 99112951.1
(22) Anmeldetag: 05.07.1999
(51) Int. Cl.: A61F 2/42

(54) **Handgelenkprothese**
Wrist prosthesis
Prothèse de poignet

(43) Veröffentlichungstag der Anmeldung: 10.01.2001
(73) Patentinhaber: Schäfer, Bernd, 6315 Oberägeri (CH)
(72) Erfinder: Hubach, Peter C.G., 1676 GH Twisk (NL); Schäfer, Bernd, 73035 Göppingen (DE); Trautwein, Thilo, 70619 Stuggart (DE)
(74) Vertreter: Dreiss, Fuhlendorf, Steimle & Becker

(56) Entgegenhaltungen:
- EP-A- 0 034 192
- EP-A- 0 454 646
- EP-A- 0 524 874
- EP-A- 0 749 735
- WO-A-92/03992
- WO-A-97/30665
- DE-A- 4 433 483
- FR-A- 2 724 310
- GB-A- 2 269 752
- US-A- 4 040 130
- US-A- 5 108 442
- US-A- 5 458 646
- US-A- 5 514 183

## Beschreibung

Die Erfindung betrifft eine Handgelenkprothese mit den Merkmalen des Oberbegriffs des Anspruchs 1.

Aus der US 5,458,646 ist eine Handgelenkprothese bekannt, bei der ein erster, proximaler Abschnitt mit dem distalen Ende der Speiche und dem distalen Ende der Elle starr verbunden ist. Ein distaler Abschnitt der Handgelenkprothese ist mit den Handwurzelknochen verbunden. Dieser zweite, distale Abschnitt der Handgelenkprothese besitzt ein abgerundetes, proximales Ende, welches an einer Geradfläche des proximalen Abschnitts anliegt. Eine derartige Handgelenkprothese hat mehrere Nachteile. Zum einen verbindet sie die Speiche mit der Elle starr, was eine Bewegung des Handgelenks in Pro- und Supinationsrichtung einschränkt. Zum anderen ist die Verbindung des distalen Abschnitts der Handgelenkprothese mit dem Handwurzelknochen unzureichend. Schließlich ist eine derartige Handgelenkprothese nur bei noch nicht weit fortgeschrittener Osteoporose und daher nur bedingt verwendbar bzw. einsetzbar.

Aus der und der WO-A-97 30 665 und der EP-A-0 034 192 ist jeweils eine Handgelenksprothese mit den Merkmalen des Oberbegriffs bekannt geworden. Die Bewegungsfreiheit ist bei dieser Prothese jedoch eingeschränkt. Aus der FR-A-2 673 100 ist ebenfalls eine Handgelenksprothese bekannt geworden, die eine eingeschränkte Bewegungsfreiheit aufweist. Dies gilt auch für die Handgelenksprothesen der DE-A-44 33 483 und der US-A-4,259,752.

Der Erfindung liegt daher die Aufgabe zugrunde, eine Handgelenkprothese bereitzustellen, die ein breiteres Einsatzgebiet aufweist.

Diese Aufgabe wird mit einer Handgelenkprothese gelöst, die die Merkmale des Anspruchs 1 aufweist.

Die erfindungsgemäße Handgelenkprothese kann vorteilhafterweise bei rheumatischer Arthritis eingesetzt werden. Sie hat den wesentlichen Vorteil, dass der proximale Abschnitt lediglich mit der Speiche starr verbunden ist. Hierfür dient ein einziger, in die Speiche einzementierter Anker, der relativ weit in die Speiche eingeführt ist. Außerdem ist der zweite, distale Abschnitt der Prothese mit wenigstens einem, insbesondere mit zwei Mittelhandknochen verbunden, wodurch eine wesentlich bessere Stabilität und Verankerung erzielt wird. Hierfür dienen Schrauben, die in die Mittelhandknochen eingeschraubt werden. Die Schrauben besitzen eine relativ lange, gewindefreie Schraubenspitze, die am vorderen Ende abgerundet ist. Durch die relativ lange Schraube wird eine sichere Verbindung des distalen Abschnitts mit den Knochen gewährleistet. Schließlich weisen die beiden Abschnitte jeweils einen Teil eines gekrümmten Lagers auf, wodurch eine optimale Abstützung sowohl in axialer Richtung als auch in allen Querrichtungen hierzu gewährleistet ist. Das gekrümmte Lager weisen mit einem der Speiche zugewandten Lagerteil und einem den Mittelhandknochen zugewandten Lagerelement auf, wobei das Lagerteil konvex und das Lagerelement konkav gekrümmt ist und die Krümmungen teilkreisförmig sind.

In bevorzugter Weise ist der Anker asymmetrisch geformt und weist einen die Speiche überragenden Ankerkopf auf. Dieser Ankerkopf bildet den einen Teil des gekrümmten Lagers.

Eine bevorzugte Ausführungsform sieht vor, dass der distale Abschnitt eine Handwurzelplatte aufweist, in welcher die Verbindungselemente zu den Handknochen, insbesondere Knochenschrauben, gelagert sind. Die Handwurzelplatte weist Aufnahmen für die Schraubenköpfe auf. Durch die Verbindung der Schrauben über eine Handwurzelplatte wird der Vorteil erzielt, dass der distale Abschnitt der Handgelenkprothese relativ stabil ausgebildet ist und dadurch die Mittelhandknochen eine optimale Unterstützung erfahren und eine steife und sichere Verbindung des distalen Abschnitts mit der Hand hergestellt wird, wodurch ein Lockern weitestgehend ausgeschlossen wird.

Zur Anpassung der Handgelenkprothese an die unterschiedlichen Größen der Handgelenke ist vorgesehen, dass die Aufnahmen im Wesentlichen länglich ausgebildet sind und jeweils wenigstens zwei Aufnahmepositionen für die Schraubenköpfe aufweisen. Über diese Aufnahmepositionen kann die Lage der Schrauben an die Lage der Mittelhandknochen angepasst werden, ohne dass hierfür unterschiedlich ausgestaltete Handwurzelplatten bereitgestellt werden müssten. Die Handwurzelplatte ist also universell einsetzbar.

Insbesondere weisen die Aufnahmen für die Schrauben die Form einer Acht auf. Somit sind unterschiedliche Positionen des Schraubenkopfs möglich, und der Abstand der Schrauben zueinander ist einstellbar.

Bei einer weiteren bevorzugten Ausgestaltung der Erfindung ist vorgesehen, dass die Achsen der Aufnahmen in der Neigungsebene des Handgelenks um einen Winkel von 5° bis 15°, insbesondere 12° gegenüber der Achse des proximalen Abschnitts geneigt ist. Das Handgelenk ist also um einen Winkel im Bereich von 5° bis 15° geneigt, wobei diese Neigungsstellung die Grundstellung darstellt.

Mit Vorzug ist die Handwurzelplatte über ein Lagerelement mit dem proximalen Abschnitt verbunden. Dieses Lagerelement weist den anderen Teil des gekrümmten Lagers auf und kann in Bezug auf seine Größe an das Handgelenk angepasst werden. Durch unterschiedliche Lagerelemente kann also die Handgelenkprothese relativ genau auf die Größe des Handgelenks eingestellt werden. Das Lagerelement ist z.B. mit einer Schraubverbindung mit der Handwurzelplatte verbunden oder das Lagerelement weist eine Schwalbenschwanzverbindung auf, über welche sie mit der Handwurzelplatte verbunden ist. Die Schwalbenschwanzverbindung kann mit einer Schnapp-Rast-Verbindung versehen sein, so dass die Ankopplung des Lagerelements an die Handwurzelplatte schnell und vor allem ohne Werkzeug möglich ist.

Eine bevorzugte Ausführungsform sieht vor, dass das Lagerelement mit einem Zapfen versehen ist, der in eine Nut des Ankerkopfes eingreift, oder umgekehrt, wodurch das Lager bezüglich der Pro- und Supinationsrichtung stabilisiert ist, wobei die Neigung des Lagers jedoch unbeeinträchtigt bleibt.

Eine Weiterbildung sieht vor, dass der Ankerkopf des Ankers an seiner dem Dorn zugewanden Seite zur Aufnahme eines Elle-Speiche-Verbindungsmittels, insbesondere einer Ellenplatte, ausgebildet ist. Falls erforderlich kann der Anker mit dieser Ellenplatte bestückt werden, wobei über diese Ellenplatte eine Verbindung der Speiche mit der Elle herstellbar ist. Diese Verbindung erfolgt dadurch, dass die Ellenplatte den Ankerkopf des Ankers seitlich überragt und eine Aufnahme für eine Ellenschraube aufweist, welche in das Ende der Elle eingeschraubt wird.

In der Aufnahme für die Ellenschraube ist ein Einsatz vorgesehen, über welchen die Ellenschraube beweglich mit der Ellenplatte verbindbar ist. Auf diese Weise wird gewährleistet, dass trotz Verbindung von Elle und Speiche eine Relativbewegung dieser Knochen nach wie vor möglich ist. Somit ist das Handgelenk in Pro- und Supinationsrichtung wesentlich flexibler.

Die Ellenschraube ist bezüglich der Ellenplatte im Einsatz verschwenkbar und/oder axial verschiebbar gelagert. Diese bewegliche Lagerung erlaubt zusätzlich eine relativ unbehinderte Drehung des Handgelenks, wobei trotz der relativ großen Freiheit der Elle diese nach wie vor abgestützt wird.

Eine verschleißfeste Lagerung wird dadurch erzielt, dass das Lagerelement und/oder der Einsatz aus Kunststoff, insbesondere aus Polyethylen bestehen. Die anderen Bauteile können aus Stahl, insbesondere aus Titan bestehen, wobei die Oberfläche mit einer Beschichtung versehen ist, die ein Anwachsen des Knochens unterstützt. Eine derartige Beschichtung ist z.B. unter der Bezeichnung HA-(hydroxy appetite)-Beschichtung bekannt.

Eine problemlose Bewegung des Handgelenks wird dadurch ermöglicht, dass das gekrümmte Lager in Pro- und Supinationsrichtung sowie in Neigungsrichtung teilkreisförmig gekrümmt ist.

Eine hohe Einsatzmöglichkeit des Handgelenks wird dadurch erzielt, dass der proximale und/oder der distale Abschnitt der Handgelenkprothese aus modular miteinander kombinierbaren Bauteilen, die ggf. unterschiedliche Größen aufweisen können, aufbaubar ist. Die einzelnen Module können vor Ort, d.h. während der Operation ausgesucht und zusammengesetzt werden. Dies können z.B. unterschiedlich dicke und/oder unterschiedlich lange Knochenschrauben, unterschiedlich große Handwurzelplatten, unterschiedlich große Lagerelemente, unterschiedlich große Ellenplatten und unterschiedlich dicke und lange Anker sowie unterschiedlich große Ellenschrauben sein.

Weitere Vorteile, Merkmale und Einzelheiten der Erfindung ergeben sich aus den Unteransprüchen sowie der nachfolgenden Beschreibung, in der unter Bezugnahme auf die Zeichnung besonders bevorzugte Ausführungsbeispiele im einzelnen beschrieben sind. Dabei können die in der Zeichnung dargestellten sowie in den Ansprüchen und in der Beschreibung erwähnten Merkmale jeweils einzeln für sich oder in beliebiger Kombination erfindungswesentlich sein. In der Zeichnung zeigen:
- Figur 1: eine perspektivische Ansicht einer bevorzugten Ausführungsform der erfindungsgemäßen Handgelenkprothese;
- Figur 2: eine Ansicht auf die Schmalseite der Handgelenkprothese gemäß Figur 1;
- Figur 3: eine Ansicht auf die Breitseite der Handgelenkprothese gemäß Figur 1;
- Figur 4: eine perspektivische Ansicht des Ankers der Handgelenkprothese;
- Figur 5: eine Seitenansicht einer zweiten Ausführungsform des Ankers;
- Figur 6: eine Draufsicht auf die Unterseite der Ellenplatte;
- Figur 7: eine parspektivische Ansicht eines mit der Ellenplatte verklipsbaren Einsatzes;
- Figur 8: einen Längsschnitt VIII - VIII durch den Einsatz gemäß Figur 7;
- Figur 9: eine Seitenansicht der Ellenschraube;
- Figur 10: eine perspektivische Ansicht der Handwurzelplatte;
- Figur 11: eine Ansicht auf eine Breitseite der Handwurzelplatte gemäß Figur 10;
- Figur 12: eine Ansicht auf eine Schmalseite der Handwurzelplatte gemäß Figur 10;
- Figur 13: eine perspektivische Ansicht des mit der Handwurzelplatte gemäß Figur 10 verbindbaren Lagerelements;
- Figur 14: eine Seitenansicht des Lagerelements; und
- Figur 15: eine Seitenansicht einer weiteren Ausführungsform des Lagerelements.

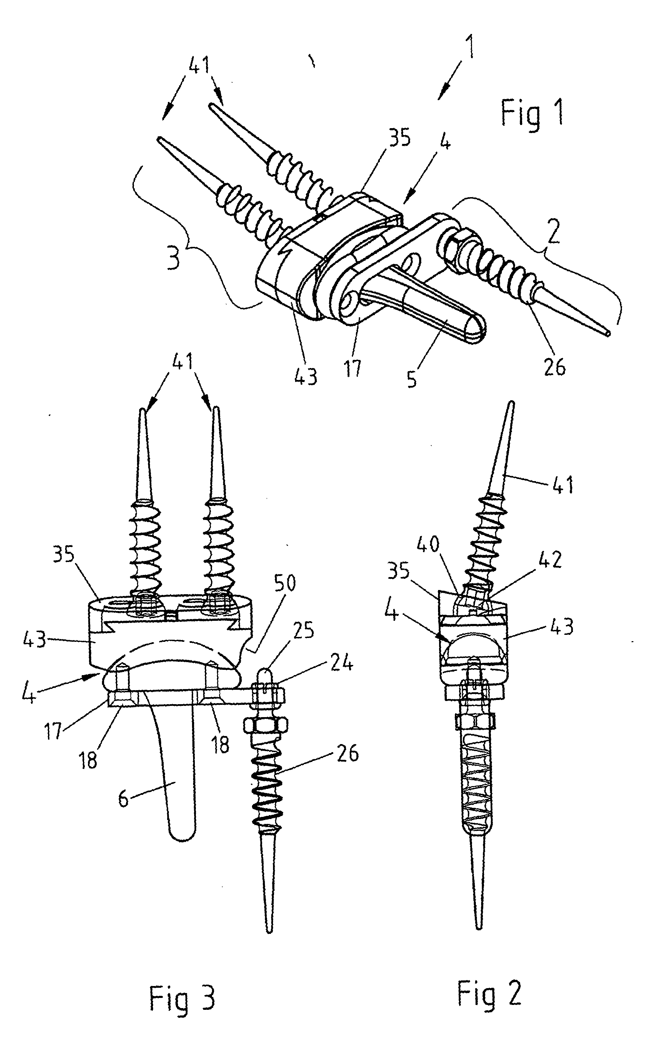

In der Figur 1 ist ein besonders bevorzugtes Ausführungsbeispiel einer insgesamt mit 1 bezeichneten Handgelenkprothese dargestellt. Diese Handgelenkprothese 1 weist einen ersten, proximalen Abschnitt 2 und einen zweiten, distalen Abschnitt 3 auf. Die beiden Abschnitte 2 und 3 sind über ein gekrümmtes Lager 4, welches in den Figuren 2 und 3 besser erkennbar ist, gelenkig miteinander verbunden.

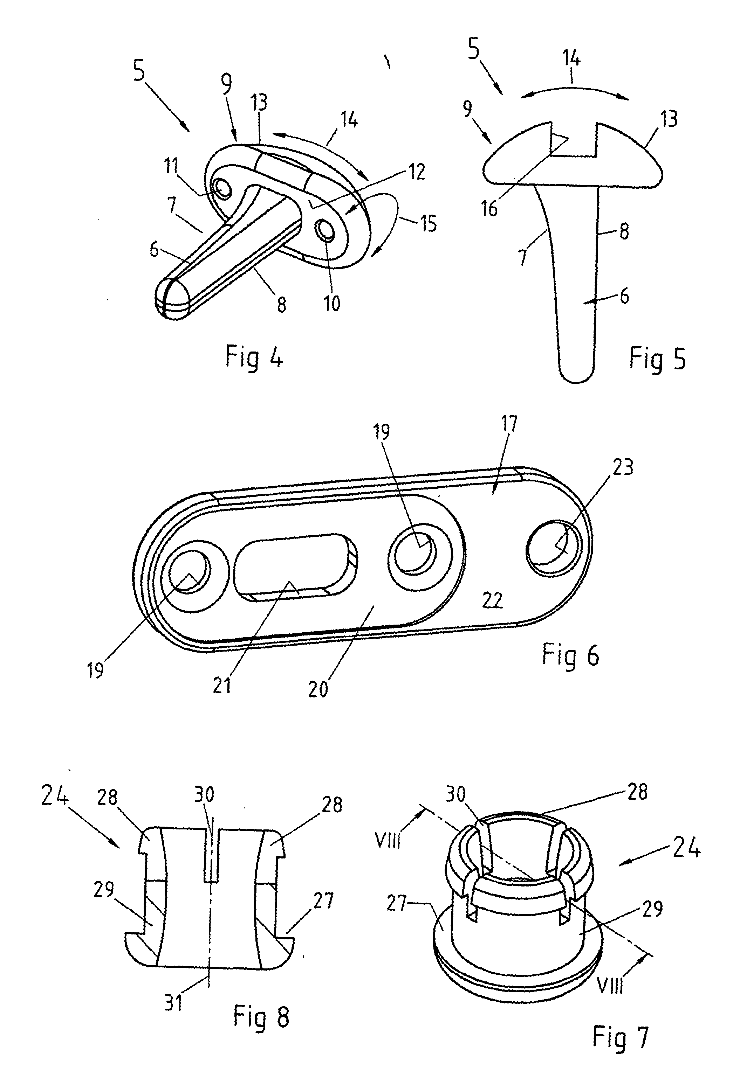

Der proximale Abschnitt 2 weist einen Anker 5 auf, der im Einzelnen in Figur 4 dargestellt ist. Der Anker 5 ist mit einem asymmetrisch ausgestalteten Dorn 6 versehen, wobei die Pronationsseite 7 gekrümmt und die Supinationsseite 8 gerade ausgebildet ist. Der Dorn 6 weist eine gerundete Spitze auf und erweitert sich in Richtung seines Ankerkopfes 9. Dieser Ankerkopf 9 weist an seiner Unterseite zwei Gewindebohrungen 10 und 11 auf, die beidseits des Dornes 8 angeordnet sind. Die Gewindebohrungen 10 und 11 sowie der Dorn 6 befinden sich in einer an der Unterseite des Ankerkopfes 9 vorgesehenen Vertiefung 12. Die Vertiefung 12 hat die Aufgabe, beim Einzementieren des Ankers 5 in die Speiche eines Unterarmes überschüssigen Zement aufzunehmen. Die dem Dorn 6 gegenüberliegende Seite des Ankerkopfes 9 bildet einen Teil 13 des gekrümmten Lagers 4, wobei dieses Lagerteil 13 sowohl in Längsrichtung (Pfeil 14) als auch in Querrichtung (Pfeil 15) teilkreisbogenförmig ausgebildet ist, wobei der Radius in Längsrichtung größer als der Radius in Querrichtung ist. Auf diese Weise wird die Möglichkeit geschaffen, dass sowohl eine Verschwenkung in Längsrichtung als auch eine Verschwenkung in Querrichtung (in Pro- und Supinationsrichtung und in Neigungsrichtung) ermöglicht, jedoch eine Verdrehung des gekrümmten Lagers 4 in sich ausgeschlossen ist.

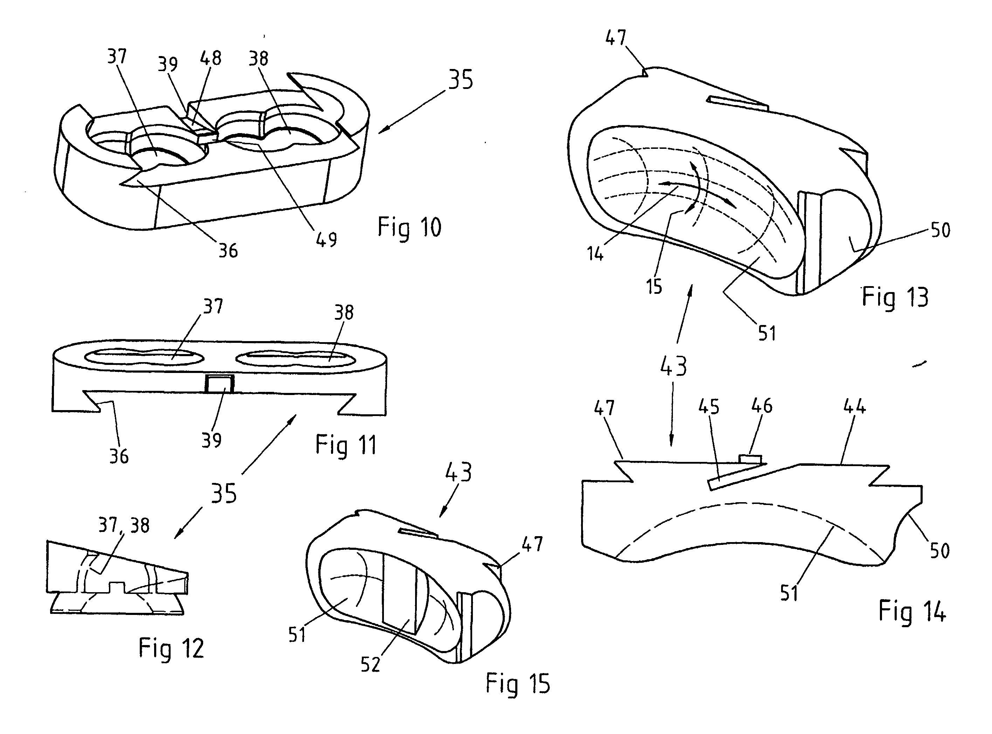

In Figur 5 ist ein zweites Ausführungsbeispiel eines Ankers 5 dargestellt, dessen Ankerkopf 9 mit einer in Querrichtung 15 verlaufenden Nut 16 versehen ist. Die Funktion der Nut 16 wird weiter unten, bei der Diskussion der Figur 15 erläutert.

Die Figur 6 zeigt eine Ellenplatte 17, die auch in den Figuren 1 bis 3 erkennbar ist. Die Ellenplatte 17 ist über Schrauben 18, deren Schraubenkopf in Senköffnungen 19 liegen, mit der Unterseite des Ankerkopfes 9, insbesondere mit den Gewindebohrungen 10 und 11 verschraubt. Die Ellenplatte 17 weist ebenfalls eine Vertiefung 20 auf, die zur Aufnahme überschüssigen Zements dient. Durch die längliche Öffnung 21 wird der Dorn 6 durch die Platte 17 geführt.

Die Ellenplatte 17 überragt den Ankerkopf 9 seitlich mit einem Abschnitt 22, der, wie dargestellt (Figuren 1 bis 3 und 6) in der gleichen Ebene der Ellenplatte 17 liegt, der jedoch bei anderen Ausführungsbeispielen (nicht dargestellt) nach unten, d.h. in Richtung des Dorns 6 abgekröpft sein kann. Die Öffnung 21 und der Abschnitt 22 befinden sich dann in zwei zueinander beabstandeten und parallelen Ebenen. Es ist jedoch auch denkbar, den Abschnitt 22 winklig gegenüber der Öffnung 21 auszugestalten.

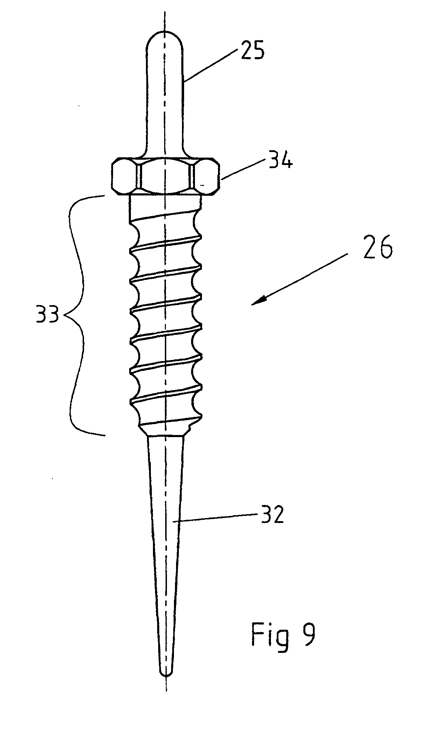

Der Abschnitt 22 weist eine Öffnung 23 auf, in welche ein Einsatz 24 einklipsbar ist. Dieser Einsatz 24 ist in den Figuren 7 und 8 dargestellt. Dieser Einsatz 24 bildet ein Lager für den zapfenartigen Kopf 25 einer Ellenschraube 26 (Figur 9). Der Einsatz 24 besitzt eine umlaufende Schulter 27, mit welcher er an der Unterseite (Figur 6) der Ellenplatte 17 anliegt. An der Oberseite der Ellenplatte 17 greifen vier elastisch federnd ausgestaltete Hakenelemente 28 an, die das Ende eines hülsenförmigen Mittelteils 29 bilden. Dieses Hülsenteil 29, dessen Ende mit vier Schlitzen 30 versehen ist, wodurch die Hakenelemente 28 gebildet werden, besitzt eine Innenoberfläche, die in Richtung der Achse 31 des Einsatzes 24 derart gekrümmt ist, dass der Öffnungsquerschnitt von den jeweiligen stirnseitigen Enden des Einsatzes 24 in Richtung des Zentrums abnimmt. Dieser Einsatz 24 besteht vorzugsweise aus einem Kunststoff, insbesondere aus Polyethylen.

Die in der Figur 9 dargestellte Ellenschraube 26 besitzt eine gewindefreie Spitze 32, einen Gewindeabschnitt 33, einen Sechskant 34 sowie den kreiszylinderförmigen Schraubenkopf 25. Die Spitze 32 ist an ihrem freien Ende abgerundet und geht kegelförmig in den Gewindeabschnitt 33 über. Der Gewindeabschnitt 33 ist ebenfalls kegelförmig ausgebildet. Über den Sechskant 34 kann die Ellenschraube 26 in eine Elle axial eingeschraubt werden, deren Kopf entfernt worden ist. Der kreiszylinderförmige Schraubenkopf 25 ist im Einsatz 24 gelagert und kann dort sowohl in Richtung der Achse 31 axial verschoben als auch um das Zentrum des Einsatzes 24 verschwenkt werden. Außerdem kann die Ellenschraube 26 um deren Längsachse im Einsatz 24 gedreht werden.

Die Figur 10 zeigt eine Handwurzelplatte 35 in perspektivischer Ansicht, wobei insbesondere eine Schwalbenschwanznut 36 sowie zwei Aufnahmen 37 und 38 und ein Teil einer Schnapp-Rast-Verbindung 39 erkennbar sind. In die Aufnahmen 37 und 38 sind die Köpfe 40 zweier Knochenschrauben 41 einsetzbar. Hierfür sind die Aufnahmen 37 und 38 kalottenförmig ausgebildet, wobei der Schraubenkopf 40 die Form eines Kugelabschnitts aufweist. Im Übrigen sind die Knochenschrauben 41 entsprechend der Ellenschraube 26 ausgebildet. Durch die kegelförmige Schraubenspitze, die gewindefrei ausgebildet ist, wird die Schraube 41 beim Eindrehen in die Mittelhandknochen optimal geführt. Zum Eindrehen wird ein Sechskantwerkzeug verwendet, welches mit der Sechskantöffnung 42 im Schraubenkopf 40 der Knochenschraube 41 zusammenwirkt.

Die Aufnahmen 37 und 38 weisen die Form einer Acht auf, wodurch die Möglichkeit geschaffen wird, dass die Knochenschrauben 41 in jeder Aufnahme 37 und 38 in zwei Position angeordnet werden kann. Auf diese Weise kann der Abstand der Knochenschrauben 41 zueinander sowie die Lage der Knochenschrauben 41 bezüglich der Handwurzelplatte 35 und somit bezüglich der Speiche optimal eingestellt werden.

Aus Figur 12 ist erkennbar, dass die Handwurzelplatte 35 eine Keilform besitzt, wobei die den Schraubenaustritt der Aufnahmen 37 und 38 aufweisende Stirnfläche bezüglich der die Schwalbenschwanznut 36 aufweisenden Grundfläche geneigt ist. Der Neigungswinkel beträgt 12°. Es besteht jedoch auch die Möglichkeit, dass die Schwalbenschwanznut 36 an einer geneigten Basisfläche vorgesehen ist, so dass die Achse der Aufnahmen 37 und 38 orthogonal zur Austrittsfläche stehen.

Die Figuren 13 und 14 zeigen ein erstes Ausführungsbeispiel eines Lagerelements 43, welches eine Schwalbenschwanzfeder 47 aufweist, die zur Schwalbenschwanznut 36 der Handwurzelplatte 35 korrespondiert. Außerdem besitzt die Oberseite 44 eine elastisch nachgiebige Zunge 45, von der ein Zapfen 46 vorspringt. Wird das Lagerelement 43 über die Schwalbenschwanzverbindung 36 und 47 mit der Handwurzelplatte 35 verbunden, dann wir der Zapfen 46 über die Einlaufschräge 48 der Schnapp-Rast-Verbindung 39 eingedrückt. Ist das Lagerelement 43 vollständig in die Handwurzelplate 35 eingeschoben, dann rastet der Zapfen 46 in die Nut 49 der Handwurzelplatte 35 ein und das Lagerelement 43 ist unverlierbar an der Handwurzelplatte 35 befestigt. Das Lagerelement 43 besteht vorzugsweise aus einem Kunststoff, insbesondere aus Polyethylen.

Die der Schwalbenschwanzfeder 47 gegenüberliegende Seite ist konkav ausgebildet und besitzt sowohl in Längsrichtung (Pfeil 14) als auch auch in Querrichtung (Pfeil 15) eine teilkreisförmige Wölbung nach innen, wobei der Radius in Längsrichtung größer ist als der Radius in Querrichtung. Die Wölbungen entsprechend den Krümmungen des Ankerkopfes 9 des Ankers 6.

Außerdem besitzt das Lagerelement 43 an einer Schmalseite eine Ausnehmung 50 (Figuren 3, 13 und 14) wodurch sichergestellt wird, dass das Lagerelement 43 beim Verschwenken in Längsrichtung (Supination) nicht mit dem Kopf 25 der Ellenschraube 26 kollidiert.

Die Figur 15 zeigt ein zweites Ausführungsbeispiel des Lagerelements 43, welches an der den zweiten Teil 51 des Lagers 4 bildenden konkaven Oberfläche einen Zapfen 52 trägt, der axial vorspringt. Dieser Zapfen 52 greift in die in der Figur 5 dargestellte Nut 16 des Ankerkopfes 9 ein. Durch diesen Zapfen 52 wird eine Bewegung des Lagers 4 in Längsrichtung (Pfeil 14) (Pro- und Supination) verhindert, wohingegen eine Neigung in Querrichtung (Pfeil 15) nach wie vor möglich ist. Auf diese Weise wird die Handgelenkprothese in einer Richtung stabilisiert.

Allgemein ist anzumerken, dass die einzelnen Bauteile abgesehen von den Elementen, über welche sie miteinander verbunden werden, unterschiedliche Größen aufweisen können. So können z.B. die Schrauben 26 und 41 unterschiedlich lang und einen unterschiedlich großen Querschnitt aufweisen, das Lagerelement 43 kann mit unterschiedlichen Höhe ausgestattet sein, die Ellenplatte 17 kann eben oder gekrümmt ausgebildet sein, wobei der seitlich vorspringende Abschnitt 22 unterschiedlich weit vorspringen kann. Außerdem kann der Anker 5 unterschiedliche Größen aufweisen.

Ein weiterer Vorteil der Erfindung besteht darin, dass die Handgelenkprothese sowohl mit als auch ohne Ellenplatte 17 verwendet werden kann.

## Patentansprüche

1. Handgelenkprothese mit einem ersten, proximalen Abschnitt (2) und einem zweiten, distalen Abschnitt (3), wobei der proximale Abschnitt (2) mit dem distalen Ende der Speiche verbunden werden kann und der distale Abschnitt (3) mit wenigstens einem, insbesondere mit zwei Mittelhandknochen verbunden werden kann und die beiden Abschnitte (2 und 3) jeweils einen Teil (13 und 51) eines gekrümmten Lagers (4) mit einem der Speiche zugewandten Lagerteil (13) und einem den Mittelhandknochen zugewandten Lagerelement (43) aufweisen, dadurch gekennzeichent, dass das Lagerteil (13) konvex und das Lagerelement (43) konkav gekrümmt ist und die Krümmungen teilkreisförmig sind.

2. Handgelenkprothese nach Anspruch 1, **dadurch gekennzeichnet, dass** der proximale Abschnitt (2) über einen in die Speiche einzementierten Anker (5) mit der Speiche verbunden ist.

3. Handgelenkprothese nach Anspruch 2, **dadurch gekennzeichnet, dass** der Anker (5) einen asymmetrisch geformten, in die Speiche einzuführenden Dorn (6) und einen das Ende der Speiche überragenden Ankerkopf (9) aufweist.

4. Handgelenkprothese nach Anspruch 3, **dadurch gekennzeichnet, dass** der Ankerkopf (9) den einen Teil (13) des gekrümmten Lagers (4) bildet.

5. Handgelenkprothese nach einem der vorhergehenden Ansprüche, **dadurch gekennzeichnet, dass** der distale Abschnitt (3) über Schrauben (41) mit den Mittelhandknochen verbunden ist.

6. Handgelenkprothese nach einem der vorhergehenden Ansprüche, **dadurch gekennzeichnet, dass** der distale Abschnitt (3) eine Handwurzelplatte (35) aufweist, in welcher die Verbindungselemente (41) zu den Mittelhandknochen gelagert sind.

7. Handgelenkprothese nach Anspruch 5 und 6, **dadurch gekennzeichnet, dass** die Handwurzelplatte (35) Aufnahmen (37, 38) für Schraubenköpfe (40) der Schrauben (41) aufweist.

8. Handgelenkprothese nach Anspruch 7, **dadurch gekennzeichnet, dass** die Aufnahmen (37, 38) im wesentlichen länglich ausgebildet sind und jeweils wenigstens zwei Aufnahmepositionen für die Schraubenköpfe (40) aufweisen.

9. Handgelenkprothese nach Anspruch 8, **dadurch gekennzeichnet, dass** die Aufnahmen (37, 38) die Form einer Acht aufweisen.

10. Handgelenkprothese nach einem der Ansprüche 7 bis 9, **dadurch gekennzeichnet, dass** die Achsen der Aufnahmen (37, 38) in der Neigungsebene des Handgelenks um einen Winkel von 5° bis 15°, insbesondere 12° gegenüber der Achse des proximalen Abschnitts (2) geneigt ist.

11. Handgelenkprothese nach einem der Ansprüche 6 bis 10, **dadurch gekennzeichnet, dass** die Handwurzelplatte (35) über ein Lagerelement (43) mit dem proximalen Abschnitt (2) verbunden ist.

12. Handgelenkprothese nach Anspruch 11, **dadurch gekennzeichnet, dass** das Lagerelement (43) ein Teil (51) des gekrümmten Lagers (4) aufweist.

13. Handgelenkprothese nach Anspruch 11 oder 12, **dadurch gekennzeichnet, dass** das Lagerelement (43) mit einem Zapfen (52) versehen ist, der in eine Nut (16) des Ankerkopfes (9) eingreift, oder umgekehrt, wodurch das Lager (4) bezüglich der Pro- und Supinationsrichtung stabilisiert ist, die Neigung des Lagers (4) aber unbeeinträchtigt bleibt.

14. Handgelenkprothese nach einem der Ansprüche 10 bis 13, **dadurch gekennzeichnet, dass** das Lagerelement (43) mittels einer Schwalbenschwanzverbindung (36, 47), insbesondere mit einer Schnapp-Rast-Verbindung (39) mit der Handwurzelplatte (35) verbunden ist.

15. Handgelenkprothese nach Anspruch 3 oder 4, **dadurch gekennzeichnet, dass** der Ankerkopf (9) des Ankers (5) an seiner dem Dorn (6) zugewandten Seite zur Aufnahme eines Elle-Speiche-Verbindngsmittels, insbesondere einer Ellenplatte (17) ausgebildet ist.

16. Handgelenkprothese nach Anspruch 15, **dadurch gekennzeichnet, dass** die Ellenplatte (17) den Ankerkopf (9) des Ankers (5) seitlich überragt und eine Aufnahme (23) für eine Ellenschraube (26) aufweist.

17. Handgelenkprothese nach Anspruch 16, **dadurch gekennzeichnet, dass** in der Aufnahme (23) für die Ellenschraube (26) ein Einsatz (24) vorgesehen ist, über welchen die Ellenschraube (26) beweglich mit der Ellenplatte (17) verbindbar ist.

18. Handgelenkprothese nach Anspruch 17, **dadurch gekennzeichnet, dass** die Ellenschraube (26) bezüglich der Ellenplatte (17) im Einsatz (24) verschwenkbar und/oder axial verschieblich gelagert ist.

19. Handgelenkprothese nach Anspruch 10 oder 11 und/oder nach Anspruch 17 oder 18, **dadurch gekennzeichnet, dass** das Lagerelement (43) und/oder der Einsatz (24) aus Kunststoff, insbesondere aus PE besteht bzw, bestehen.

20. Handgelenkprothese nach einem der vorhergehenden Ansprüche, **dadurch gekennzeichnet, dass** das gekrümmte Lager (4) in Pro- und Supinationsrichtung und in Neigungsrichtung teilkreisförmig gekrümmt ist.

21. Handgelenkprothese nach einem der vorhergehenden Ansprüche, **dadurch gekennzeichnet, dass** der proximale und/oder der distale Abschnitt (2, 3) aus modular miteinander kombinierbaren Bauteilen, die gegebenenfalls unterschiedliche Größen aufweisen können, aufbaubar ist.

## Claims

1. Wrist prosthesis with a first, proximal section (2) and a second, distal section (3), wherein the proximal section (2) can be connected with the distal end of the radius and the distal section (3) can be connected with at least one, especially with two, metacarpal bones, and each of the sections (2 and 3) contains one part (13 and 51) of a curved bearing, having a bearing part (13) facing the radius and having a bearing element (43) facing the metacarpal bones, **characterized in that** the bearing part (13) has a convex and the bearing element (43) has a concave curvature and that the curvatures are partially circular.

2. Wrist prosthesis according to claim 1, **characterized in that** the proximal section (2) is connected with the radius by an anchor (5) cemented in the radius.

3. Wrist prosthesis according to claim 2, **characterized in that** the anchor (5) has an asymmetrically-shaped pin (6) to be guided into the radius and an anchor head (9) extending above the end of the radius.

4. Wrist prosthesis according to claim 3, **characterized in that** the anchor head (9) forms the one part (13) of the curved bearing (4).

5. Wrist prosthesis according to one of the preceding claims, **characterized in that** the distal section (3) is connected with the metacarpal bones by way of screws (41).

6. Wrist prosthesis according to one of the preceding claims, **characterized in that** the distal section (3) has a carpal plate, in which the elements (41) connecting to the metacarpal bones are journaled.

7. Wrist prosthesis according to claims 5 and 6, **characterized in that** the carpal plate (35) has seats (37, 38) for the screw heads (40) of the screws (41).

8. Wrist prosthesis according to claim 7, **characterized in that** the seats (37, 38) are essentially longitudinally expanded and each has at least two seating positions for the screw heads (40).

9. Wrist prosthesis according to claim 8, **characterized in that** the seats (37, 38) have the form of a figure 8.

10. Wrist prosthesis according to one of the claims 7 to 9, **characterized in that** the axes of the seats (37, 38) are inclined in the inclination plane of the wrist by an angle from 5° to 15°, especially 12°, relative to the axis of the proximal section (2).

11. Wrist prosthesis according to claims 6 to 10, **characterized in that** the carpal plate (35) is connected with the proximal section (2) by way of a bearing element (43).

12. Wrist prosthesis according to claim 11, **characterized in that** the bearing element (43) has one part (51) of the curved bearing (4).

13. Wrist prosthesis according to claim 10 or 12, **characterized in that** the bearing element (43) is provided with a lug (52), which extends into a groove (16) of the anchor head (9), or vice versa, so that the bearing (4) is stabilized relative to the pronation- and supination direction, while inclination of the bearing (4) remains unaffected.

14. Wrist prosthesis according to one of the claims 10 to 13, **characterized in that** the bearing element (43) is connected with the carpal plate (35) by means of a dovetail connection (36, 47), especially with a snap-lock connection (39).

15. Wrist prosthesis according to claim 3 or 4, **characterized in that** the anchor head (9) of the anchor (5) is formed on its side facing the pin (6) with an area for receiving an ulna-radius connecting means, especially an ulna plate (17).

16. Wrist prosthesis according to claim 15, **characterized in that** the ulna plate (17) extends laterally beyond the anchor head (9) of the anchor (5) and has a seat (23) for an ulna screw (26).

17. Wrist prosthesis according to claim 16, **characterized in that** an insert (24) is provided in the seat (23) for the ulna screw (26) and the ulna screw (26) is movably connectable with the ulna plate (17) by way of such insert.

18. Wrist prosthesis according to claim 17, **characterized in that** the ulna screw (26) is journaled in the insert (24) such that it can swivel and/or undergo axial displacement relative to the ulna plate (17).

19. Wrist prosthesis according to claim 10 or 11 and/or according to claim 17 or 18, **characterized in that** the bearing element (43) and/or the insert (24) are, or is, made of plastics material, especially PE.

20. Wrist prosthesis according to one of the preceding claims, **characterized in that** the curved bearing (4) has partially circular arc curvature in the pronation- and supination direction and in the inclination direction.

21. Wrist prosthesis according to one of the preceding claims, **characterized in that** the proximal and/or distal section (2, 3) can be assembled from modularly combinable components, which may have different sizes.

## Revendications

1. Prothèse de poignet formée d'une première portion proximale (2) et d'une seconde portion distale (3), la portion proximale (2) pouvant être reliée à l'extrémité distale du radius, la portion distale (3) pouvant être reliée à au moins un et en particulier deux métacarpiens et chacune des deux portions (2 et 3) présentant une partie (13 et 51) d'un organe d'articulation incurvé (4) composé d'une partie d'articulation (13) en regard du radius et d'un élément d'articulation (43) en regard des métacarpiens, **caractérisée en ce que** la partie d'articulation (13) et l'élément d'articulation (43) sont respectivement incurvés selon des formes convexe et concave.

2. Prothèse de poignet selon la revendication 1, **caractérisée en ce que** la portion proximale (2) est reliée au radius par un élément d'ancrage (5) scellé dans le radius au moyen de ciment.

3. Prothèse de poignet selon la revendication 2, **caractérisée en ce que** l'élément d'ancrage (5) présente une tige (6) de forme asymétrique, destinée à être introduite dans le radius, et une tête d'ancrage (9) qui dépasse de l'extrémité du radius.

4. Prothèse de poignet selon la revendication 3, **caractérisée en ce que** la tête d'ancrage (9) forme la partie (13) de l'organe d'articulation incurvé (4).

5. Prothèse de poignet selon l'une quelconque des revendications précédentes, **caractérisée en ce que** la portion distale (3) est reliée aux métacarpiens par des vis (41).

6. Prothèse de poignet selon l'une quelconque des revendications précédentes, **caractérisée en ce que** la portion distale (3) présente une plaque carpienne (35), dans laquelle sont montés les éléments de liaison (41) aux métacarpiens.

7. Prothèse de poignet selon les revendications 5 et 6, **caractérisée en ce que** la plaque carpienne (35) présente des logements (37, 38) destinés à recevoir les têtes (40) des vis (41).

8. Prothèse de poignet selon la revendication 7, **caractérisée en ce que** les logements (37, 38) sont de forme générale oblongue et présentent chacun au moins deux positions de logement pour les têtes de vis (40).

9. Prothèse de poignet selon la revendication 8, **caractérisée en ce que** les logements (37, 38) affectent la forme d'un huit.

10. Prothèse de poignet selon l'une quelconque des revendications 7 à 9, **caractérisée en ce que** l'axe des logements (37, 38) dans le plan d'inclinaison de poignet est incliné de 5° à 15° et en particulier de 12° par rapport à l'axe de la portion proximale (2).

11. Prothèse de poignet selon l'une quelconque des revendications 6 à 10, **caractérisée en ce que** la plaque carpienne (35) est reliée à la portion proximale (2) par un élément d'articulation (43).

12. Prothèse de poignet selon la revendication 11, **caractérisée en ce que** l'élément d'articulation (43) présente une partie (51) de l'organe d'articulation incurvé (4).

13. Prothèse de poignet selon la revendication 11 ou 12, **caractérisée en ce que** l'élément d'articulation (43) présente un ressaut (52) qui s'engage dans une rainure (16) de la tête d'ancrage (9), ou vice versa, assurant ainsi la stabilisation de l'organe d'articulation (4) dans la direction de pronation et de supination, l'inclinaison de l'organe d'articulation (4) demeurant toutefois libre.

14. Prothèse de poignet selon l'une quelconque des revendications 10 à 13, **caractérisée en ce que** l'élément d'articulation (43) est relié à la plaque carpienne (35) au moyen d'un assemblage à queue d'aronde (36, 47), en particulier un assemblage par encliquetage (39).

15. Prothèse de poignet selon la revendication 3 ou 4, **caractérisée en ce que** la tête d'ancrage (9) de l'élément d'ancrage (5) est formée, sur sa face en regard de la tige (6), pour recevoir un moyen de liaison radio-cubital, en particulier une plaque cubitale (17).

16. Prothèse de poignet selon la revendication 15, **caractérisée en ce que** la plaque cubitale (17) dépasse latéralement de la tête d'ancrage (9) de l'élément d'ancrage (5) et présente un logement (23) destiné à recevoir une vis cubitale (26).

17. Prothèse de poignet selon la revendication 16, **caractérisée en ce que** pour recevoir la vis cubitale (26), il est prévu dans le logement (23) un insert (24) permettant de relier avec capacité de mouvement la vis cubitale (26) à la plaque cubitale (17).

18. Prothèse de poignet selon la revendication 17, **caractérisée en ce que** la vis cubitale (26) est montée dans l'insert (24) avec capacité de pivotement et/ou de translation axiale par rapport à la plaque cubitale (17).

19. Prothèse de poignet selon la revendication 10 ou 11 et/ou selon la revendication 17 ou 18, **caractérisée en ce que** l'élément d'articulation (43) et/ou l'insert (24) sont réalisés en matière plastique, en particulier en PE.

20. Prothèse de poignet selon l'une quelconque des revendications précédentes, **caractérisée en ce que** l'organe d'articulation incurvé (4) est incurvé sur un secteur de cercle dans la direction de pronation et de supination et dans la direction d'inclinaison.

21. Prothèse de poignet selon l'une quelconque des revendications précédentes, **caractérisée en ce que** la portion proximale (2) et/ou la portion distale (3) peuvent être formées de composants aptes à être combinés entre eux de manière modulaire et susceptibles de présenter des tailles différentes.
